# EUROPEAN PATENT APPLICATION

(11) **EP 4 116 289 A1**
(43) Date of publication of application: **11.01.2023**
(21) Application number: 20922355.1
(22) Date of filing: 31.03.2020
(51) Int. Cl.: C07C 319/20, C07C 323/25, C07C 211/29, C07C 231/12, C07C 233/78, C07C 213/02, C07C 217/26, C07C 211/15, C07C 211/35, C07D 209/34, C07D 307/33, C07D 235/14, C07D 213/38, C12N 9/10, C12P 13/00, C12P 17/12

(54) **SYNTHESIS METHOD FOR FLUORINE-CONTAINING CHIRAL AMINE COMPOUND**

(30) Priority: 26.02.2020 CN 202010118880
(71) Applicant: Asymchem Laboratories (Tianjin) Co., Ltd., Tianjin 300457 (CN)
(72) Inventor: HONG, Hao, Morrisville North Carolina 27560 (US); JAMES, Gage, Morrisville, North Carolina 27560 (US); XIAO, Yi, Tianjin 300457 (CN); ZHANG, Na, Tianjin 300457 (CN); LI, Xiang, Tianjin 300457 (CN); JIANG, Xiangjun, Tianjin 300457 (CN); LV, Shunxing, Tianjin 300457 (CN)
(74) Representative: Berggren Oy
(86) International application number: PCT/CN2020/082595
(87) International publication number: WO 2021/168988

(57) **Abstract**

The present invention provides a method for synthesizing a fluorine-containing chiral amine compound. The method comprised: reacting an amino donor with a fluorine-containing dihydroxy ketal compound under a catalysis of a transaminase to generate the fluorine-containing chiral amine compound, wherein the transaminase is derived from a plurality of strains. The transaminase of the present application has substrate specificity on the fluorine-containing dihydroxy ketal compound, and may effectively catalyze this type of the substrate to be converted into the fluorine-containing chiral amine compound. In addition, the transaminase has catalytic activity on a plurality of the fluorine-containing dihydroxy ketal compounds, and is relatively high in reaction selectivity and activity. The method catalyzed by the bio-enzyme is not only short in route, but also high in product yield, and the production using the method reduces cost, organic solvents and three wastes.

## Description

### Cross-reference to related applications

This application is based on Chinese Patent Application No. 202010118880.8, filed February 26, 2020, which claims the benefit of priority to the Chinese Patent Application, which is incorporated by reference in its entirety herein.

### Technical Field

The present invention relates to the field of synthesis of fluorine-containing compounds, in particular to a method for synthesizing a fluorine-containing chiral amine compound.

### Background

Fluorine-containing compounds are a very important type of compounds in medicine and agricultural chemistry. In the past decade, fluoride-containing drugs are grown at an unprecedented rate. Therefore, the development of a green and efficient synthesis method plays a crucial role. Fluorine-containing dihydroxy ketal compounds are also one of this type of the compounds 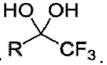 Important fluorine-containing chiral amine compounds 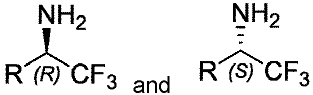 may be synthesized by this type of substrates. At present, methods for synthesizing fluorine-containing chiral amines by fluorine-containing dihydroxy ketal are mainly as follows.
1. According to a reaction route shown in a Formula 1: under an alkaline condition, a C-C bond of a fluorine-containing dihydroxy ketal compound 1 is easily cleaved, to form a trifluoroacetic acid and a corresponding fluorine-containing enol compound 2. A compound 3 is used as a Mannich reaction acceptor, a compound 4 is obtained after a Mannich reaction, and then a sulfinyl group is removed to obtain a target product, a fluorine-containing chiral amine compound. 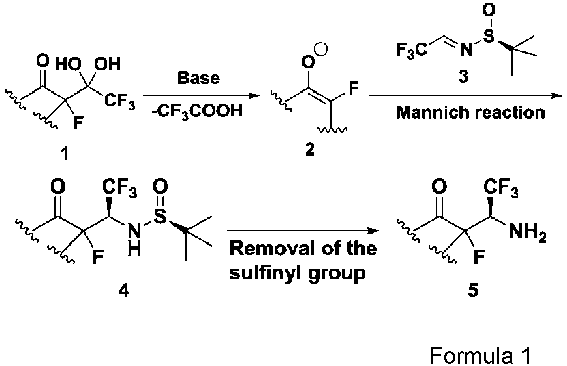
2. According to a reaction route shown in a Formula 2: a fluorine-containing dihydroxy ketal compound 6 is reacted with a benzylamine compound, a chiral amino group is introduced, and then a benzyl group is removed to obtain a target compound 8.

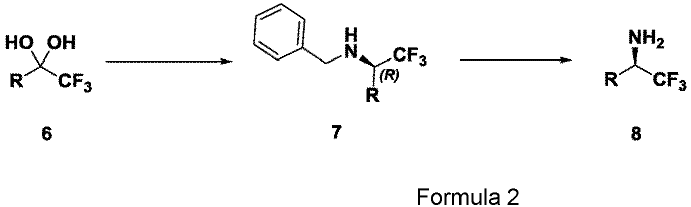

The substrate of the route shown in the above the Formula 1 has limitations, and the target product may be obtainedafter a three-step reaction, and the yield is low. The reaction route is longer, and chemical reagents used in the reaction produce a large amount of three wastes. The route shown in the Formula 2 is a two-step reaction, but the reaction sometimes requires the use of expensive metal ligands or catalysts, as well as the use of highly toxic compounds. Similarly, the use of chemical reagents in the reaction produces a large amount of the three wastes.

Therefore, there is still a need to provide a new synthesis method for such fluorine-containing dihydroxy ketal compounds.

### Summary

A main purpose of the present invention is to provide a method for synthesizing a fluorine-containing chiral amine compound, as to solve a problem in an existing technology that the fluorine-containing chiral amine compound is long in synthesis route.

In order to achieve the above purpose, the present invention provides a method for synthesizing a fluorine-containing chiral amine compound, wherein the method comprises: reacting an amino donor with a fluorine-containing dihydroxy ketal compound under the catalysis of a transaminase to generate the fluorine-containing chiral amine compound, wherein the transaminase is derived from Chromobacterium violaceum DSM30191(CVTA)(NCBI Reference Sequence: WP_011135573.1), Fonsecaea pedrosoi CBS 271.37(NCBI Reference Sequence: XP_013286281.1), Klebsiella pneumoniae subsp.pneumoniae EcI8(GenBank: CCN29541.1), Mycobacterium goodie(GenBank: AKS36000.1), Paracoccus denitrificans(NCBI Reference Sequence:WP_011746975.1), Penicillium brasilianum(GenBank: CEJ55334.1), Enterobacter sp.TL3(NCBI Reference Sequence: WP_014885677.1), Aspergillus terreusNIH2624(NCBI Reference Sequence: XP_001209325.1), Exophiala spinifera(NCBI Reference Sequence: XP_016233821.1), Deinococcus geothermalis(strain DSM 11300)(NCBI Reference Sequence: WP_011530545.1), Geomyces destructans 20631-21(GdTA)in E.coli(GenBank: ELR05573.1), Pseudomonas putida KT2440(NCBI Reference Sequence: WP_010954554.1), Lysinibacillus sphaericus(NCBI Reference Sequence: WP_024363741.1), Bacillus megaterium DSM 319(NCBI Reference Sequence: WP_013082219.1), Trichoderma harzianum(GenBank: KKP07030.1), Aspergillus fumigatus R-ATAs(AspFum)(NCBI Reference Sequence: XP_748821.1), Geobacillus thermodenitrificans subsp.thermodenitrificans DSM 465(NCBI Reference Sequence: WP_008879436.1), Cladophialophora bantiana CBS 173.52(NCBI Reference Sequence: XP_016617948.1), Bacillus megaterium(NCBI Reference Sequence: WP_016763026.1), Burkholderia thailandensis MSMB121(BtS-TA)(GenBank: AGK49399.1), Klebsiella pneumoniae subsp.pneumoniae MGH 78578(NCBI Reference Sequence: WP_002920226.1), Geobacillus toebii(NCBI Reference Sequence: WP_062753894.1) and Talaromyces cellulolyticus(GenBank: GAM37533.1).

Further, the transaminase has an amino acid sequence shown in SEQ ID NO: 1, SEQ ID NO: 2, SEQ ID NO: 3 or SEQ ID NO: 4.

Further, the fluorine-containing dihydroxy ketal compound has a structural as shown in formula I:

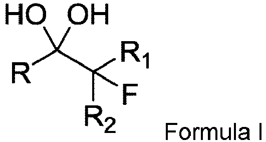

Herein, R is selected from an alkyl, a cycloalkyl, an aryl, alkyl containing one or more heteroatoms, a cycloalkyl containing one or more heteroatoms, an aryl containing one or more heteroatoms, an amide compound residue or an ether compound residue, and R1 and R2 are the same or different hydrogen atoms or halogen atoms.

More preferably, the fluorine-containing dihydroxy ketal compound has a structural as shown in formula II:

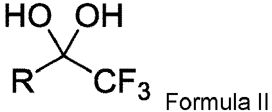

Further, the fluorine-containing dihydroxy ketal compound is selected from any one of the followings. 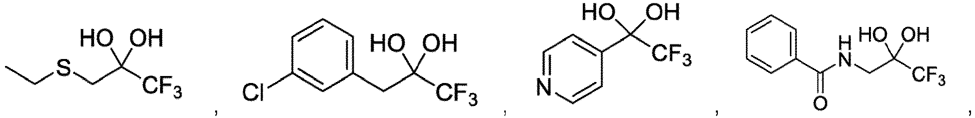 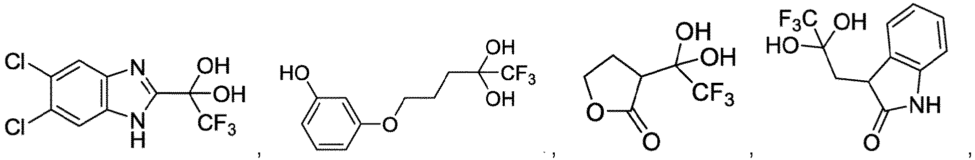 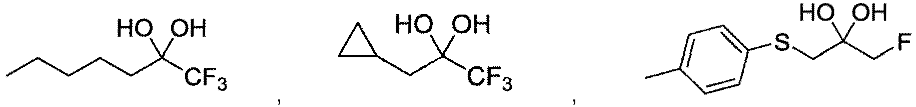 and

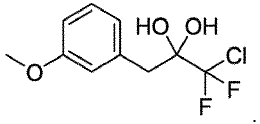

Further, the method includes: mixing a phosphate buffer with the amino donor to obtain a first mixed solution, adding the fluorine-containing dihydroxy ketal compound to the first mixed solution to obtain a second mixed solution; adding the transaminase to the second mixed solution to obtain a reaction mixture; and isolating the fluorine-containing chiral amine compound from the reaction mixture.

Further, before adding the fluorine-containing dihydroxy ketal compound to the first mixed solution, the method further includes: adjusting a pH value of the first mixed solution to 7.0-9.0; and preferably, adding the transaminase to the second mixed solution, and adjusting pH of the second mixed solution after adding the transaminase to 7.0-9.0, to obtain the reaction mixture.

Further, isolating the fluorine-containing chiral amine compound from the reaction mixture comprises: adjust the acidity of the reaction mixture to obtain a denatured transaminase, and preferably adjusting a pH value of the reaction mixture to 1-2; filtering and removing the denatured transaminase, to obtain filtrate; extracting the filtrate, to obtain the fluorine-containing chiral amine compound; preferably, the filtering is carried out with dichloromethane; and preferably, the extracting is carried out with the dichloromethane.

Further, the extraction is carried out 2-5 times, and after the extractions, a step of drying organic phase resulting from the extractions is further included. Preferably, combining the organic phases obtained from the extractions to obtain an extract; drying the extract to obtain a dried organic phase of product; and concentrating the dried organic phase of product to no fraction under a temperature less than 35°C and a pressure no more than -0.06 Mpa to obtain the fluorine-containing chiral amine compound.

Further, a mass ratio of the transaminase to the fluorine-containing dihydroxy ketal compound is 0.4: 1-1.0: 1.

Further, a concentration of the fluorine-containing dihydroxy ketal compound is 60 g/L~100 g/L.

Further, the amino donor is selected from isopropylamine, a hydrochloride of the isopropylamine, alanine, aniline or n-butylamine.

A technical scheme of the present invention is applied, the transaminase of the present application has substrate specificity on the fluorine-containing dihydroxy ketal compound, and may effectively catalyze this type of the substrate to be converted into the fluorine-containing chiral amine compound. In addition, the transaminase has catalytic activity on a plurality of the fluorine-containing dihydroxy ketal compounds, and is relatively high in reaction selectivity and activity. The method catalyzed by the bio-enzyme is not only short in route, but also high in product yield, and the production using the method reduces cost, organic solvents and three wastes.

### Brief Description of the Drawings

Drawings of the description constituting a part of the present application are used to provide further understanding of the present invention, and exemplary embodiments of the present invention and descriptions thereof are used to explain the present invention, and do not constitute improper limitation to the present invention. In the drawings:
Fig. 1 shows effects of different enzyme amounts on the conversion rate of the same amount of a substrate in Embodiment 13 of the present invention.
Fig. 2 shows effects of the same enzyme amount on the conversion rate of the substrates with different concentrations in Embodiment 14 of the present invention.
Fig. 3 shows effects of different amino donors on the conversion rate of the substrate in the same reaction in Embodiment 15 of the present invention.
Fig. 4 shows that transaminases from different sources in Embodiment 16 of the present invention all have conversion activities for the same substrate.

### Detailed Description of the Embodiments

It should be noted that embodiments in the present application and features of the embodiments may be combined with each other in the case without conflicting. The present invention is described in detail below in combination with the embodiments.

As mentioned in the background, in an existing technology, while the fluorine-containing dihydroxy ketal compound is used as a starting raw material to synthesize the fluorine-containing chiral amine compound, there is not only a defect of a long reaction route, but also a problem of three industrial wastes. In order to improve this situation, the inventor of the present application tries to improve an existing synthesis route from the perspective of high efficiency andenvironmental protection. In the research process, it is found by the inventor that a transaminase developed by the applicant himself not only has the general catalytic activity of the transaminase for catalyzing ketone compounds with a pyridoxal phosphate (PLP) as a coenzyme, but also has the activity of catalyzing the fluorine-containing dihydroxy ketal compound. In addition, it is found from the further research that while the transaminase is used to catalyze a fluorine-containing dihydroxy ketal compound substrate, not only a target product may be obtained by an one-step reaction, but also the substrate type is wide. Due to the high selectivity of a biotransformation reaction using the transaminase, an enantiomeric excess (ee) value of the target product is greatly improved. Moreover, the use of a biological enzyme to catalyze the reaction greatly improves the production efficiency and reduces the generation of organic solvents and three wastes.

On the basis of the above research results, the applicant proposes a technical scheme of the present application. In a typical embodiment of the present application, a method for synthesizing a fluorine-containing chiral amine compound is provided, and the method includes: reacting an amino donor with a fluorine-containing dihydroxy ketal compound under a catalysis of a transaminase to generate the fluorine-containing chiral amine compound, herein the transaminase is derived from Chromobacterium violaceum DSM30191(CVTA)(NCBI Reference Sequence: WP_011135573.1), Fonsecaea pedrosoi CBS 271.37(NCBI Reference Sequence: XP_013286281.1), Klebsiella pneumoniae subsp.pneumoniae EcI8(GenBank: CCN29541.1), Mycobacterium goodii(GenBank: AKS36000.1), Paracoccus denitrificans(NCBI Reference Sequence: WP_011746975.1), Penicillium brasilianum(GenBank: CEJ55334.1), Enterobacter sp.TL3(NCBI Reference Sequence: WP_014885677.1), Aspergillus terreusNIH2624(NCBI Reference Sequence: XP_001209325.1), Exophiala spinifera(NCBI Reference Sequence: XP_016233821.1), Deinococcus geothermalis(strain DSM 11300)(NCBI Reference Sequence: WP_011530545.1), Geomyces destructans 20631-21(GdTA)in E.coli(GenBank: ELR05573.1), Pseudomonas putida KT2440(NCBI Reference Sequence: WP_010954554.1), Lysinibacillus sphaericus(NCBI Reference Sequence: WP_024363741.1), Bacillus megaterium DSM 319(NCBI Reference Sequence: WP_013082219.1), Trichoderma harzianum(GenBank: KKP07030.1), Aspergillus fumigatus R-ATAs(AspFum)(NCBI Reference Sequence: XP_748821.1), Geobacillus thermodenitrificans subsp.thermodenitrificans DSM 465(NCBI Reference Sequence: WP_008879436.1), Cladophialophora bantiana CBS 173.52(NCBI Reference Sequence: XP_016617948.1), Bacillus megaterium(NCBI Reference Sequence: WP_016763026.1), Burkholderia thailandensis MSMB121(BtS-TA)(GenBank: AGK49399.1), Klebsiella pneumoniae subsp.pneumoniae MGH 78578(NCBI Reference Sequence: WP_002920226.1), Geobacillus toebii(NCBI Reference Sequence: WP_062753894.1) and Talaromyces cellulolyticus(GenBank: GAM37533.1).

More preferably, the transaminase has an amino acid sequence shown below in SEQ ID NO: 1, SEQ ID NO: 2, SEQ ID NO: 3 or SEQ ID NO: 4:

MQKQRTCSQWRELDAAHHLHPFTDTASLNQAGARVMTRGEGVYLWDCEGNKIIDGMAGLW
CVNVGYGRKDFAEAARRQMEELPFYNTFYKTTHPAVVELSSLLAEVTPAGFDRVFYTNSGSESVD
TMIRMVRRYWDVQGKPEKKTLIGRWWGYHGSTIGGASLGGFKYMHEQGDLPIPGMAHIEQPWW
YKHGKDMTPDEFGVVAARWLEEKILEIGADKVAAFVGEPIQGAGGVIVPPATYWPEIERICRKYDVL
LVADEVICGFGRTGEWFGHQHFGFQPDLFTAAKGLSSGYLPIGAVFVGKRVAEGLIAGGDFNHGFT
YSGHPVCAAVAHANVAALRDEGIVQRVKDDIGPYMQKRWRETFSRFEHVDDVRGVGMLLAFTLVK
NKAKRELFPDFGEIGTLCRDIFFRNNLIMDSCGDHIVCAPPLVMTRAEVDEMLAVAERCLEEFEQTL
KARGLA (SEQ ID NO: 1)。

MQKQRTCSQWRELDAAHHLHPFTDTASLNQAGARVMTRGEGVYLWDCEGNKIIDGMAGLW
CVNVGYGRKDFAEAARRQMEELPFYNTFYKTTHPAVVELSSLLAEVTPAGFDRVFYTNSGSESVD
TMIRMVRRYWDVQGKPEKKTLIGRWWGYHGSTIGGASLGGFKYMHEQGDLPIPGMAHIEQPWW
YKHGKDMTPDEFGVVAARWLEEKILEIGADKVAAFVGEPIQGAGGVIVPPATYWPEIERICRKYDVL
LVADEVICGFGRTGEWFGHQHFGFQPDLFTAAKGLSSGYLPIGAVFVGKRVAEGLIAGGDFNHGFT
YSGHPVCAAVAHANVAALRDEGIVQRVKDDIGPYMQKRWRETFSRFEHVDDVRGVGMLLAFTLVK
NKAKRELFPDFGEIGTLCRDIFFRNNLIMDSCGDHIVAAPPLVMTRAEVDEMLAVAERCLEEFEQTL
KARGLA (SEQ ID NO: 2)。

MQKQRTCSQWRELDAAHHLHPFTDTASLNQAGARVMTRGEGVYLWDCEGNKIIDGMAGLW

CVNVGYGRKDFAEAARRQMEELPFYNTFYKTTHPAVVELSSLLAEVTPAGFDRVFYTNSGSESVD
TMIRMVRRYWDVQGKPEKKTLIGRWWGYHGSTIGGASLGGFKYMHEQGDLPIPGMAHIEQPWW
YKHGKDMTPDEFGVVAARWLEEKILEIGADKVAAFVGEPIQGAGGVIVPPATYWPEIERICRKYDVL
LVADEVICGFGRTGEWFGHQHFGFQPDLFTAAKGLSSGYLPIGAVFVGKRVAEGLIAGGDFNHGFT
YSGHPVCAAVAHANVAALRDEGIVQRVKDDIGPYMQKRWRETFSRFEHVDDVRGVGMLLAFTLVK
NKAKRELFPDFGEIGTLCRDIFQRNNLIMDSCGDHIVSAPPLVMTRAEVDEMLAVAERCLEEFEQTL
KARGLA (SEQ ID NO： 3)。

MQKQRTCSQWRELDAAHHLHPFTDTASLNQAGARVMTRGEGVYLWDCEGNKIIDGMAGLW
CVNVGYGRKDFAEAARRQMEELPFYNTFYKTTHPAVVELSSLLAEVTPAGFDRVFYTNSGSESVD
TMIRMVRRYWDVQGKPEKKTLIGRWWGYHGSTIGGASLGGFKYMHEQGDLPIPGMAHIEQPWW
YKHGKDMTPDEFGVVAARWLEEKILEIGADKVAAFVGEPIQGAGGVIVPPATYWPEIERICRKYDVL
LVADEVICGFGRTGEWFGHQHFGFQPDLFTAAKGLSSGYLPIGAVFVGKRVAEGLIAGGDFNHGFT
YSGHPVCAAVAHANVAALRDEGIVQRVKDDIGPYMQKRWRETFSRFEHVDDVRGVGMLLAFTLVK
NKAKRELFPDFGEIGTLCRDIFHRNNLIMDSCGDHIVSAPPLVMTRAEVDEMLAVAERCLEEFEQTL
KARGLA (SEQ ID NO： 4)。

As described above, the above transaminase of the present application has substrate specificity on the fluorine-containing dihydroxy ketal compound, and may effectively catalyze this type of the substrate to be converted into the fluorine-containing chiral amine compound. In addition, the transaminase has catalytic activity on a plurality of the fluorine-containing dihydroxy ketal compounds, and also has relatively highreaction selectivity and activity. The bio-enzyme catalytic synthesis method is not only short in route, but also high in product yield, the production cost is greatly reduced, and the production of organic solvents and three wastes is reduced.

The transaminase shown in any one of the above SEQ ID NO: 1 to 4 is a ω-transaminase, and all are derived from Chromobacterium violaceum DSM30191 (CVTA). It is separated from Chromobacterium violaceum DSM30191 (CVTA) according to an existing method.

In a preferred embodiment, the fluorine-containing dihydroxy ketal compound has a structure shown in the following formula I:

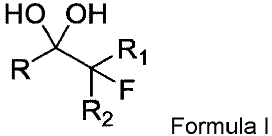

Herein, R is selected from an alkyl, a cycloalkyl, an aryl, an alkyl containing one or more heteroatoms, a cycloalkyl containing one or more heteroatoms, an aryl containing one or more heteroatoms, an amide compound residue or an ether compound residue, and R1 and R2 are the same or different hydrogen or halogen atoms.

In a more preferred embodiment, both R1 and R2 are fluorine, namely the fluorine-containing dihydroxy ketal compound has a structure shown in the following structural formula II:

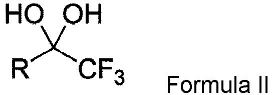

In a preferred embodiment, the fluorine-containing dihydroxy ketal compound is selected from any one of the followings. 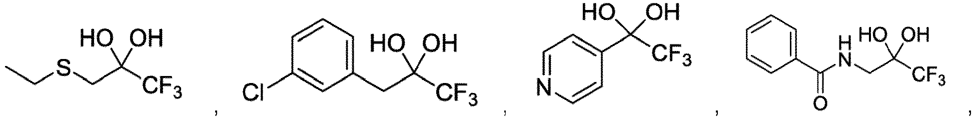 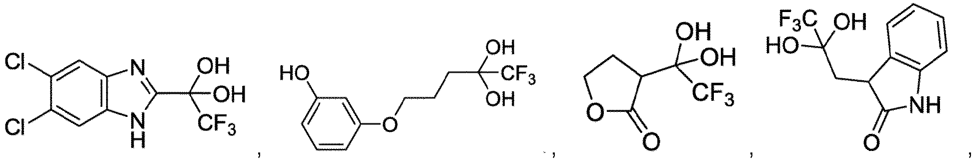 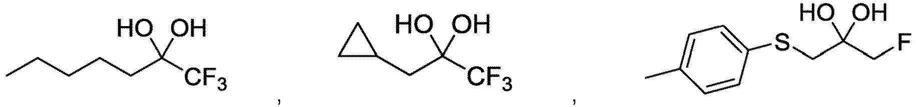 and

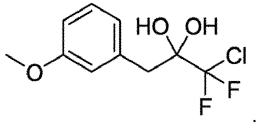

The transaminase of the present application has the catalytic activity for the above fluorine-containing dihydroxy ketal compound.

A specific step of using the transaminase of the present application to catalyze a transamination reaction of the fluorine-containing dihydroxy ketal compound is similar to an existing transamination reaction. In a preferred embodiment, the method includes: mixing a phosphate buffer with the amino donor, to obtain a first mixed solution. Adding the fluorine-containing dihydroxy ketal compound to the first mixed solution, to obtain a second mixed solution, Adding the transaminase to the second mixed solution, to obtain a reaction mixture. And isolating the fluorine-containing chiral amine compound from the reaction mixture.

The synthesis method reduces the reaction volume by the above step-by-step reaction (a previous material may be changed in order, and the last addition of the transaminase is to ensure that the reaction is performed immediately after the addition, to prevent the inactivation of the transaminase due to a longer time of other operations), so that the transaminase is relatively high in catalytic activity and efficiency on the substrate, thereby the higher product yield is obtained, and the production efficiency is improved.

In a preferred embodiment, before adding the fluorine-containing dihydroxy ketal compound to the first mixed solution, the method further comprises: adjusting a pH value of the first mixed solution to 7.0-9.0; and preferably, adding the transaminase to the second mixed solution, and adjusting pH of the second mixed solution after adding the transaminase to 7.0-9.0, to obtain the reactionmixture.

In the above preferred embodiment, before adding the fluorine-containing dihydroxy ketal compound to the first mixed solution, the pH value of the first mixed solution is adjusted. Its purpose is to adjust to the optimum pH of the enzyme reaction before adding the enzyme, and prevent the inactivation of the enzyme in the adding process. After the transaminase is added to the second mixed solution, the pH value of the system is also adjusted, its function is to ensure that the reaction is performed at the optimum pH. It is better to keep the same range of pH value adjustment for the above two times, for example, the pH value is previously adjusted to 7.8, and it is also adjusted to 7.8 after the transaminase is added.

The above step of separating, which obtains the target product from the mixed solution after the reaction, may be performed by using a known separation and purification method. In a preferred embodiment, wherein isolatingthe fluorine-containing chiral amine compound from the reaction mixture comprises: adjust the acidity of the reaction mixture to denature the transaminase, and preferably adjusting a pH value of the reaction mixture to 1-2; filtering and removing the denatured transaminase, to obtain a filtrate; extracting the filtrate, to obtain the fluorine-containing chiral amine compound; preferably, filtering is carried out with dichloromethane; and preferably, extracting is carried out with the dichloromethane.

A mode of adjusting the acidity is used to denature a reacted enzyme protein. Compared with other denaturation modes (such as high temperature, alkali adjustment or salting out), it has advantages that the denaturation is more thorough, most of products are stable under an acidic condition, and a subsequent operation may directly extract other impurities in the system under the acidic condition. The extraction with the dichloromethanehas the advantage of higher extraction efficiency compared to the extraction with other extraction solvents (such as ethyl acetate, methyl tert-butyl ether, or isopropyl acetate).

In order to further improve the purity and yield, in a preferred embodiment, the extraction is carried out 2-5 times, and after the extraction, a step of drying organic phase is further included. Preferably, combining the organic phases obtained from the extrations to obtain an extract; drying the extract to obtain a dried organic phase of product; concentrating the dried organic phase of product to no fraction under a temperature less than 35°C and a pressure no matter than -0.06 Mpa to obtain the fluorine-containing chiral amine compound (a purpose of drying is to reduce the water content in the product, and the pressure affects the concentration rate).

In a preferred embodiment, the mass ratio of the transaminase to the fluorine-containing dihydroxy ketal compound is 0.4: 1-1.0: 1, and the conversion rate of the substrate under this mass ratio reaches more than 95%.

In a preferred embodiment, the concentration of the fluorine-containing dihydroxy ketal compound is 60 g/L~100 g/L. In this concentration range, the use of the biological enzyme for the reaction not only increase the conversion rate of the substrate to 99%, but also helps to improve the concentration of the substrate, thereby the production efficiency is improved, and the generation of organic solvents and three wastes is reduced.

In a preferred embodiment, the amino donor is selected from isopropylamine, hydrochloride of the isopropylamine, alanine, aniline or n-butylamine. The conversion rates of the above amino-donating substrates may all reach more than 90%.

The beneficial effects of the present application are further described below in combination with specific embodiments. It should be noted that the room temperature in the following embodiments refers to a temperature within the normal temperature range of 10~25°C, and the transaminase used in the following Embodiments 1 to 15 is the transaminase shown in SEQ ID NO: 4.

### Embodiment 1

50 mL of 100 mmol/L phosphate buffer (5 vol) and 32 mL of 5 mol/L isopropylamine hydrochloride solution (3.2 vol, 3 eq) are added to a 250 mL four-neck flask at the room temperature, and it is adjusted that pH=7.5~8.0. Then 0.1 g of pyridoxal phosphate (1wt%), and 10 g of 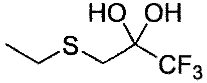 are added, and stirred uniformly, then 10 ml of transaminase enzyme solution (0.5 wt, 0.5 g/ml) is added, and adjust the pH to7.5∼8.0. The temperature is raised to 29°C and it is stirred and reacted overnight. After the reaction is completed, the system is adjusted to pH=1, as to denature a protein. After being filtered, filtrate is extracted with 50 mL of dichloromethane. Aqueous phase is adjusted to pH=13, and extracted for three times with 50 ml of dichloromethane. Organic phases are combined and dried with an anhydrous magnesium sulfate, then the organic phase is concentrated until there is no fraction under conditions of T<35°C and P≤-0.06Mpa. A target product 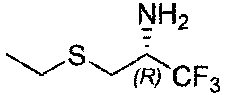 is obtained.

It is detected by a high performance liquid chromatography (HPLC), purity: 98%, ee value: 100%, yield: 87%.

### Embodiment 2

50 mL of 100 mmol/L phosphate buffer (5 vol) and 25 mL of 5 mol/L isopropylamine hydrochloride solution (2.5 vol, 3 eq) are added to a 250 mL four-neck flask at the room temperature, and it is adjusted that pH=8.0~8.5. Then 0.1 g of pyridoxal phosphate (1wt%), and 10 g of 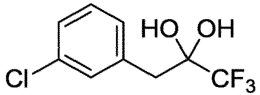 are added, and stirred uniformly, then 10 ml of transaminase enzyme solution (0.5 wt, 0.5 g/ml), and adjust the pH to8.0~8.5. The temperature is raised to 20°C and it is stirred and reacted overnight. After the reaction is completed, the system is adjusted to pH=2, as to denature a protein. After being filtered, filtrate is extracted with 50 mL of dichloromethane. Aqueous phase is adjusted to pH=13, and extracted for three times with 50 ml of ethyl acetate. Organic phases are combined and dried with an anhydrous magnesium sulfate, then the organic phase is concentrated until there is no fraction under conditions of T<45°C and P≤-0.08Mpa. A target product 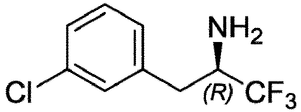 is obtained.

It is detected by HPLC, purity: 99%, ee value: 100%, yield: 86%.

### Embodiment 3

50 mL of 100 mmol/L phosphate buffer (5 vol) and 31 mL of 5 mol/L isopropylamine hydrochloride solution (3.1 vol, 3 eq) are added to a 250 mL four-neck flask at the room temperature, and it is adjusted that pH=7.0~7.5. Then 0.1 g of pyridoxal phosphate (1wt%), and 10 g of 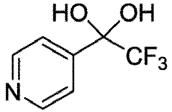 are added, and stirred uniformly, then 10 ml of transaminase enzyme solution (0.5 wt, 0.5 g/ml) is added. The temperature is raised to 45~50°C and it is stirred and reacted overnight. After the reaction is completed, the system is adjusted to pH=1, as to denature a protein. After being filtered, filtrate is extracted with 50 mL of dichloromethane. Aqueous phase is adjusted to pH=12, and extracted for three times with 50 ml of dichloromethane. Organic phases are combined and dried with an anhydrous magnesium sulfate, then the organic phase is concentrated until there is no fraction under conditions of T<35°C and P≤-0.06Mpa. A target product 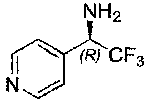 is obtained.

It is detected by HPLC, purity: 97%, ee value: 100%, yield: 83%.

### Embodiment 4

50 mL of 100 mmol/L phosphate buffer (5 vol) and 24 mL of 5 mol/L isopropylamine hydrochloride solution (2.4 vol, 3 eq) are added to a 250 mL four-neck flask at the room temperature, and it is adjusted that pH=8.5~9.0. Then 0.1 g of pyridoxal phosphate (1wt%), and 10 g of 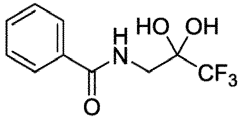 are added, and stirred uniformly, then 10 ml of transaminase enzyme solution (0.5 wt, 0.5 g/ml), and adjust the pH to 8.5~9.0. The temperature is raised to 10°C and it is stirred and reacted overnight. After the reaction is completed, the system is adjusted to pH=1, as to denature a protein. After being filtered, filtrate is extracted with 50 mL of dichloromethane. Aqueous phase is adjusted to pH=13, and extracted for three times with 50 ml of dichloromethane. Organic phases are combined and dried with an anhydrous magnesium sulfate, then the organic phase is concentrated until there is no fraction under conditions of T<35°C and P≤-0.06Mpa. A target product 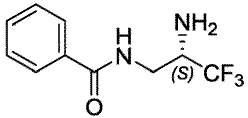 is obtained.

It is detected by HPLC, purity: 98%, ee value: 100%, yield: 90%.

### Embodiment 5

50 mL of 100 mmol/L phosphate buffer (5 vol) and 20 mL of 5 mol/L isopropylamine hydrochloride solution (2 vol, 3 eq) are added to a 250 mL four-neck flask at the room temperature, and it is adjusted that pH=7.5~8.0. Then 0.1 g of pyridoxal phosphate (1wt%), and 10 g of 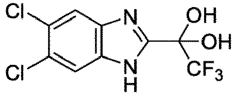 are added, and stirred uniformly, then 10 ml of transaminase enzyme solution (0.5 wt, 0.5 g/ml), and adjust the pH to 7.5~8.0. The temperature is raised to 32°C and it is stirred and reacted overnight. After the reaction is completed, the system is adjusted to pH=1, as to denature a protein. After being filtered, filtrate is extracted with 50 mL of dichloromethane. Aqueous phase is adjusted to pH=13, and extracted for three times with 50 ml of ethyl acetate. Organic phases are combined and dried with an anhydrous magnesium sulfate, then the organic phase is concentrated until there is no fraction under conditions of T<45°C and P≤-0.08Mpa. A target product 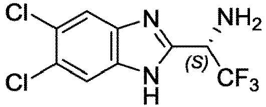 is obtained.

It is detected by HPLC, purity: 98%, ee value: 100%, yield: 87%.

### Embodiment 6

50 mL of 100 mmol/L phosphate buffer (5 vol) and 23 mL of 5 mol/L isopropylamine hydrochloride solution (2.3 vol, 3 eq) are added to a 250 mL four-neck flask at the room temperature, and it is adjusted that pH=7.0~7.5. Then 0.1 g of pyridoxal phosphate (1wt%), and 10 g of 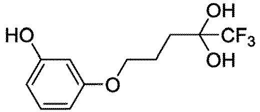 are added, and stirred uniformly, then 10 ml of transaminase enzyme solution (0.5 wt, 0.5 g/ml), and adjust the pH to pH=7.0~7.5. The temperature is raised to 28°C and it is stirred and reacted overnight. After the reaction is completed, the system is adjusted to pH=1, as to denature a protein. After being filtered, filtrate is extracted with 50 mL of dichloromethane. Aqueous phase is adjusted to pH=12, and extracted for three times with 50 ml of dichloromethane. Organic phases are combined and dried with an anhydrous magnesium sulfate, then the organic phase is concentrated until there is no fraction under conditions of T<35°C and P≤-0.06Mpa. A target product 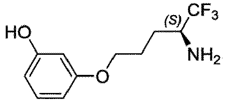 is obtained.

It is detected by HPLC, purity: 98%, ee value: 100%, yield: 80%.

### Embodiment 7

200 mL of 100 mmol/L phosphate buffer (10 vol) and 60 mL of 5 mol/L isopropylamine hydrochloride solution (3 vol, 3 eq) are added to a 500 mL four-neck flask at the room temperature, and it is adjusted that pH=7.5~8.0. Then 0.2 g of pyridoxal phosphate (1wt%), and 20 g of 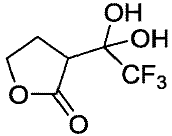 are added, and stirred uniformly, then 20 ml of transaminase enzyme solution (0.5 wt, 0.5 g/ml), and adjust the pH to 7.5~8.0. The temperature is raised to 35°C and it is stirred and reacted overnight. After the reaction is completed, the system is adjusted to pH=1, as to denature a protein. After being filtered, filtrate is extracted with 100 mL of dichloromethane. Aqueous phase is adjusted to pH=12, and extracted for three times with 100 ml of dichloromethane. Organic phases are combined and dried with an anhydrous magnesium sulfate, then the organic phase is concentrated until there is no fraction under conditions of T<35°C and P≤-0.06Mpa. A target product 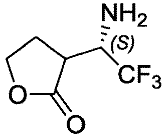 is obtained.

It is detected by HPLC, purity: 94%, ee value: 100%, yield: 82%.

### Embodiment 8

80 mL of 100 mmol/L phosphate buffer (8 vol) and 23 mL of 5 mol/L isopropylamine hydrochloride solution (2.3 vol, 3 eq) are added to a 250 mL four-neck flask at the room temperature, and it is adjusted that pH=7.0~7.5. Then 0.1 g of pyridoxal phosphate (1wt%), and 10 g of 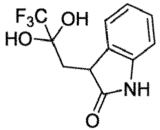 are added, and stirred uniformly, then 20 ml of transaminase enzyme solution (1.0 wt, 0.5 g/ml), and adjust the pH to 7.0~7.5. The temperature is raised to 35°C and it is stirred and reacted overnight. After the reaction is completed, the system is adjusted to pH=1, as to denature a protein. After being filtered, filtrate is extracted with 50 mL of dichloromethane. Aqueous phase is adjusted to pH=12, and extracted for three times with 50 ml of dichloromethane. Organic phases are combined and dried with an anhydrous magnesium sulfate, then the organic phase is concentrated until there is no fraction under conditions of T<35°C and P≤-0.06Mpa. A target product 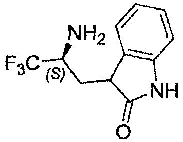 is obtained.

It is detected by HPLC, purity: 97%, ee value: 100%, yield: 86%.

### Embodiment 9

50 mL of 100 mmol/L phosphate buffer (10 vol) and 16 mL of 5 mol/L isopropylamine hydrochloride solution (3.2 vol, 3 eq) are added to a 250 mL four-neck flask at the room temperature, and it is adjusted that pH=7.5~8.0. Then 0.05 g of pyridoxal phosphate (1wt%), and 5 g of 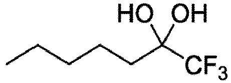 are added, and stirred uniformly, then 10 ml of transaminase enzyme solution (1.0 wt, 0.5 g/ml), and adjust the pH to. The temperature is raised to 20°C and it is stirred and reacted overnight. After the reaction is completed, the system is adjusted to pH=1, as to denature a protein. After being filtered, filtrate is extracted with 30 mL of dichloromethane. Aqueous phase is adjusted to pH=12, and extracted for three times with 30 ml of dichloromethane. Organic phases are combined and dried with an anhydrous magnesium sulfate, then the organic phase is concentrated until there is no fraction under conditions of T<35°C and P≤-0.06Mpa. A target product 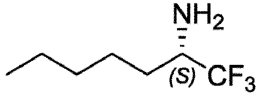 is obtained.

It is detected by a gas chromatography (GC), purity: 95%, ee value: 100%, yield: 78%.

### Embodiment 10

50 mL of 100 mmol/L phosphate buffer (10 vol) and 18 mL of 5 mol/L isopropylamine hydrochloride solution (3.6 vol, 3 eq) are added to a 250 mL four-neck flask at the room temperature, and it is adjusted that pH=7.5~8.0. Then 0.05 g of pyridoxal phosphate (1wt%), and 5 g of 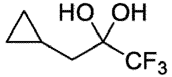 are added, and stirred uniformly, then 10 ml of transaminase enzyme solution (1.0 wt, 0.5 g/ml), and adjust the pH to. The temperature is raised to 25°C and it is stirred and reacted overnight. After the reaction is completed, the system is adjusted to pH=1, as to denature a protein. After being filtered, filtrate is extracted with 40 mL of dichloromethane. Aqueous phase is adjusted to pH=12, and extracted for three times with 40 ml of dichloromethane. Organic phases are combined and dried with an anhydrous magnesium sulfate, then the organic phase is concentrated until there is no fraction under conditions of T<35°C and P≤-0.06Mpa. A target product 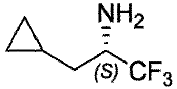 is obtained.

It is detected by GC, purity: 94%, ee value: 100%, yield: 76%.

### Embodiment 11

50 mL of 100 mmol/L phosphate buffer (10 vol) and 14 mL of 5 mol/L isopropylamine hydrochloride solution (2.8 vol, 3 eq) are added to a 250 mL four-neck flask at the room temperature, and it is adjusted that pH=7.5~8.0. Then 0.05 g of pyridoxal phosphate (1wt%), and 5 g of 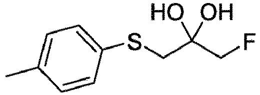 are added, and stirred uniformly, then 10 ml of transaminase enzyme solution (1.0 wt, 0.5 g/ml), and adjust the pH to. The temperature is raised to 25°C and it is stirred and reacted overnight. After the reaction is completed, the system is adjusted to pH=1, as to denature a protein. After being filtered, filtrate is extracted with 40 mL of dichloromethane. Aqueous phase is adjusted to pH=12, and extracted for three times with 40 ml of dichloromethane. Organic phases are combined and dried with an anhydrous magnesium sulfate, then the organic phase is concentrated until there is no fraction under conditions of T<35°C and P≤-0.06Mpa. A target product 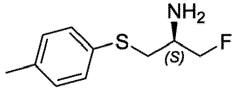 is obtained.

It is detected by HPLC, purity: 92%, ee value: 100%, yield: 79%.

### Embodiment 12

60 mL of 100 mmol/L phosphate buffer (12 vol) and 13 mL of 5 mol/L isopropylamine hydrochloride solution (2.6 vol, 3 eq) are added to a 250 mL four-neck flask at the room temperature, and it is adjusted that pH=7.5~8.0. Then 0.05 g of pyridoxal phosphate (1wt%), and 5 g of 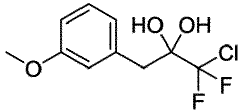 are added, and stirred uniformly, then 10 ml of transaminase enzyme solution (1.0 wt, 0.5 g/ml), and adjust the pH to. The temperature is raised to 35°C and it is stirred and reacted overnight. After the reaction is completed, the system is adjusted to pH=1, as to denature a protein. After being filtered, filtrate is extracted with 40 mL of dichloromethane. Aqueous phase is adjusted to pH=12, and extracted for three times with 40 ml of dichloromethane. Organic phases are combined and dried with an anhydrous magnesium sulfate, then the organic phase is concentrated until there is no fraction under conditions of T<35°C and P≤-0.06Mpa. A target product 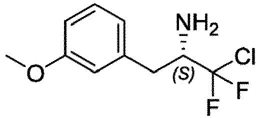 is obtained.

It is detected by HPLC, purity: 95%, ee value: 100%, yield: 84%.

### Embodiment 13

Embodiment 1 is taken as an example, the enzyme amount of the reaction is optimized and a specific operation is as follows: 1 mL of 100 mmol/L phosphate buffer (5 vol), and 0.64 ml of 5 mol/L isopropylamine hydrochloride solution (3.2 vol, 3eq) are added to a 10 mL vial at a room temperature, it is adjusted that pH=7.5~8.0. Then 0.002 g of pyridoxal phosphate (1wt%), and 0.2 g of 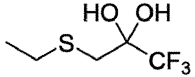 are added, and mixed uniformly, then 0.04 ml, 0.08 ml, 0.12 ml, 0.16 ml, 0.2 ml, 0.24 ml, 0.28 ml, 0.32 ml, 0.36 ml, 0.4 ml, 0.44 ml, 0.48 ml, 0.52 ml, 0.56 ml, and 0.6 ml (the concentration of the enzyme solution is 0.5 g/ml, the corresponding mass ratios are 0.1 wt, 0.2 wt, 0.3 wt, 0.4 wt, 0.5 wt, 0.6 wt, 0.7 wt, 0.8 wt, 0.9 wt, 1.0 wt, 1.1 wt, 1.2 wt, 1.3 wt, 1.4 wt, and 1.5 wt) of transaminase enzyme solution are respectively added, and adjust the pH to7.5∼8.0. The rotation speed of a shaker is 170 rpm, and the temperature is raised to 29°C and it is reacted overnight.

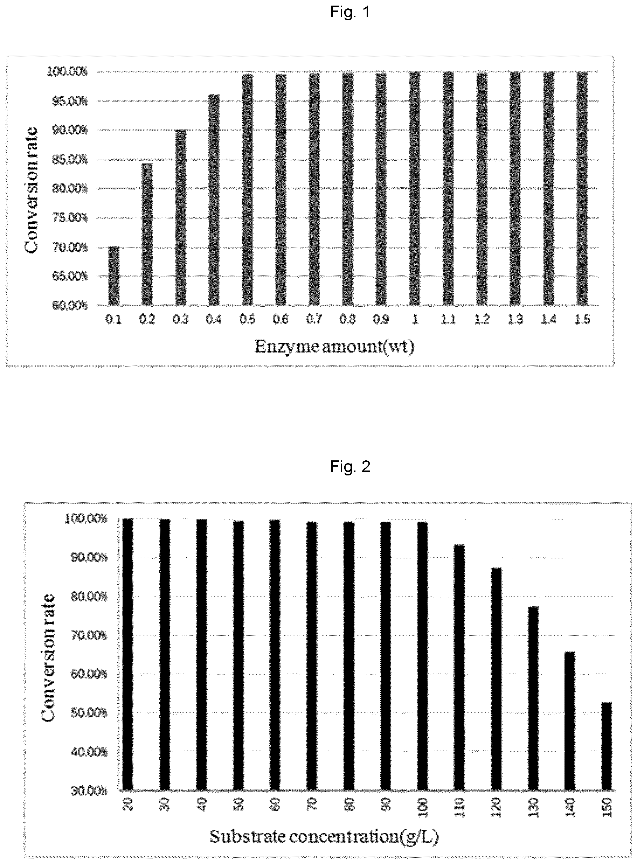

Conversion rate results are shown in Fig. 1, and it is proved that the conversion rate of 0.5~1.0 wt substrate is greater than 99%, and there is no significant effect while the enzyme amount continues to increase.

### Embodiment 14

Embodiment 1 is taken as an example, the substrate concentration is optimized and a specific operation is as follows: 0.3 mL, 0.38 mL, 0.50 mL, 0.62 mL, 0.78 mL, 0.96 ml, 1.16 ml, 1.46 ml, 1.82 ml, 2.28 ml, 2.96 ml, 3.96 ml, 5.56 ml, and 8.96 ml of 100 mmol/L phosphate buffer (1.5 vol, 1.9 vol, 2.5 vol, 3.1 vol, 3.9 vol, 4.8 vol, 5.8 vol, 7.3 vol, 9.1 vol, 11.4 vol, 14.8 vol, 19.8 vol, 27.8 vol, and 44.8 vol respectively), and 0.64 ml of 5 mol/L isopropylamine hydrochloride solution (3.2 vol, 3 eq) are respectively added to a 10-50 mL vial(an appropriate shake flask is selected according to the reaction volume) at the room temperature, it is adjusted that pH =7.5~8.0. Then 0.002 g of pyridoxal phosphate (1wt%), and 0.2 g of 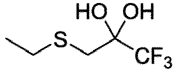 are added, and mixed uniformly, then 0.2 ml of transaminase enzyme solution (0.5 wt, the enzyme solution concentration is 0.5 g/ml), and adjust the pH to 7.5~8.0. The rotation speed of a shaker is 170 rpm, and the temperature is raised to 29°C and it is reacted overnight.

Conversion rate results are shown in Fig. 2, and it is proved that the substrate conversion rate is greater than 99% at the concentration of 60-100 g/L. While the substrate concentration continues to decrease, the reaction volume may be increased, and the amount of three wastes is increased. There is no significant effect.

### Embodiment 15

Embodiment 1 is taken as an example, the amino donor is optimized and a specific operation is as follows: 1 ml of 100 mmol/L phosphate buffer (5 vol) is added to a 10-50 mL vial (an appropriate shake flask is selected according to the reaction volume) at the room temperature, and systems of 3 eq amino donors (the amino donors are isopropylamine, isopropylamine hydrochloride, alanine, aniline, and n-butylamine respectively) are used respectively, and it is adjusted that pH=7.5~8.0. Then 0.002 g of pyridoxal phosphate (1wt%), and 0.2 g of 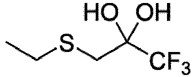 are added, and mixed uniformly, then 0.2 ml of transaminase enzyme solution (0.5 wt, the enzyme solution concentration is 0.5 g/ml), and adjust the pH to7.5∼8.0. The rotation speed of a shaker is 170 rpm, and the temperature is raised to 29°C and it is reacted overnight.

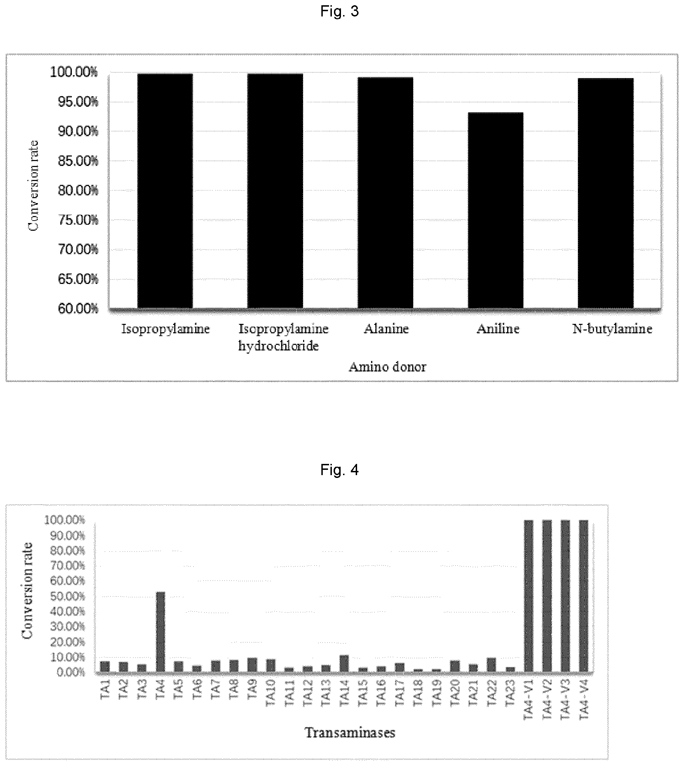

Conversion rate results are shown in Fig. 3, and it is proved that the isopropylamine, isopropylamine hydrochloride, alanine, and n-butylamine are used as the amino donors, and the substrate conversion rate is greater than 99%. While the aniline is used as the amino donor, the conversion rate is 93% under this reaction condition.

### Embodiment 16

The substrate of Embodiment 1 is taken as an example, the transaminase derived from Mycobacterium goodii (GenBank: AKS36000.1) is numbered as TA1, the transaminase derived from Fonsecaea pedrosoi CBS 271.37 (NCBI Reference Sequence: XP_013286281.1) is numbered as TA2, the transaminase derived from Klebsiella pneumoniae subsp.pneumoniae Ecl8 (GenBank: CCN29541.1) is numbered as TA3, the transaminase derived from Chromobacterium violaceum DSM30191 (CVTA) (NCBI Reference Sequence: WP_011135573.1) is numbered as TA4, the transaminase derived from Paracoccus denitrificans (NCBI Reference Sequence: WP_011746975.1) is numbered as TA5, the transaminase derived from Penicillium brasilianum (GenBank: CEJ55334.1) is numbered as TA6, the transaminase derived from Enterobacter sp.TL3 (NCBI Reference Sequence: WP_014885677.1) is numbered as TA7, the transaminase derived from Aspergillus terreus NIH2624 (NCBI Reference Sequence: XP_001209325.1) is numbered as TA8, the transaminase derived from Exophiala spinifera (NCBI Reference Sequence: XP_016233821.1) is numbered as TA9, the transaminase derived from Deinococcus geothermalis (strain DSM 11300) (NCBI Reference Sequence: WP_011530545.1) is numbered as TA10, the transaminase derived from Geomyces destructans 20631-21 (GdTA) in E. coli (GenBank: ELR05573.1) is numbered as TA11, the transaminase derived from Pseudomonas putida KT2440 (NCBI Reference Sequence: WP_010954554.1) is numbered as TA12, the transaminase derived from Lysinibacillus sphaericus (NCBI Reference Sequence: WP_024363741.1) is numbered as TA13, the transaminase derived from Bacillus megaterium DSM 319 (NCBI Reference Sequence: WP_013082219.1) is numbered as TA14, the transaminase derived from Trichoderma harzianum (GenBank: KKP07030.1) is numbered as TA15, the transaminase derived from Aspergillus fumigatus R-ATAs (AspFum) (NCBI Reference Sequence: XP_748821.1) is numbered as TA16, the transaminase derived from Geobacillus thermodenitrificans subsp.thermodenitrificans DSM 465 (NCBI Reference Sequence: WP_008879436.1) is numbered as TA17, the transaminase derived from Cladophialophora bantiana CBS 173.52 (NCBI Reference Sequence: XP_016617948.1) is numbered as TA18, the transaminase derived from Bacillus megaterium (NCBI Reference Sequence: WP_016763026.1) is numbered as TA19, the transaminase derived from Burkholderia thailandensis MSMB121 (BtS-TA) (GenBank: AGK49399.1) is numbered as TA20, the transaminase derived from Klebsiella pneumoniae subsp.pneumoniae MGH 78578 (NCBI Reference Sequence: WP_002920226.1) is numbered as TA21, the transaminase derived from Geobacillus toebii (NCBI Reference Sequence: WP_062753894.1) is numbered as TA22, the transaminase derived from Talaromyces cellulolyticus (GenBank: GAM37533.1) is numbered as TA23, mutants TA4-V1 (a corresponding sequence is SEQ ID NO: 1), TA4-V2 (a corresponding sequence is SEQ ID NO: 2), TA4-V3 (a corresponding sequence is SEQ ID NO: 3), and TA4-V4 (a corresponding sequence is SEQ ID NO: 4) obtained by evolution of the transaminase derived from Chromobacterium violaceum DSM30191 (CVTA) (NCBI Reference Sequence: WP_011135573.1) perform a 10 mg-level screening reaction, and an operation is as follows.

100 mmol/L phosphate buffer and 5 mol/L isopropylamine hydrochloride solution are prepared into 50 ml of solution according to a volume ratio of 5: 2, and then 0.005 g of pyridoxal phosphate is added, and it is stirred and mixed uniformly. 0.3 ml of the above solution is taken and added to a 96-well plate respectively, and 10 mg of 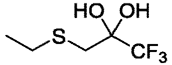 is added, and then 0.2 ml of the above enzyme solution (10 wt, the concentration of the enzyme solution is 0.5 g/ml) is added respectively, the rotation speed of a shaker is 170 rpm, the temperature is raised to 30°C and it is reacted overnight.

Screening results are shown in Fig. 4: it is proved that all the enzymes from all sources have the catalytic activity on the substrate, the transaminase derived from Chromobacterium violaceum DSM30191 (CVTA) is the best, and the transaminase derived from Chromobacterium violaceum DSM30191 (CVTA) is mutated, and four mutants obtained have the better conversion rates, and the conversion rates are all greater than 99%.

From the above descriptions, it may be seen that the above embodiments of the present invention achieve the following technical effects.
1. It is not found yet that there is a patent for converting the similar compound with an invertase.
2. The biotransformation reaction using the transaminase may directly obtain the desired target compound by the one-step reaction, and the substrate types are widely applicable.
3. The use of the transaminase for this biotransformation reaction has the high selectivity, and the ee value of the product is greatly improved.
4. The self-extracted transaminase is used, the substrate concentration may reach 100 g/L, the production efficiency is greatly improved, and the generation of the organic solvents and three wastes is reduced.
5. The transaminase is high in catalytic efficiency; and the reaction which uses the transaminase is small in reaction volume, short in synthesis route, and has high product yield; and the production cost is greatly reduced.

The above are only preferred embodiments of the present invention, and are not intended to limit the present invention. For those skilled in the art, the present invention may have various modifications and changes. Any modifications, equivalent replacements, improvements and the like made within the spirit and principle of the present invention shall be included within a scope of protection of the present invention.

## Claims

1. A method for synthesizing a fluorine-containing chiral amine compound, wherein the method comprises:
reacting an amino donor with a fluorine-containing dihydroxy ketal compound under a catalysis of a transaminase to generate the fluorine-containing chiral amine compound;
wherein the transaminase is derived from Chromobacterium violaceum DSM30191(CVTA), Fonsecaea pedrosoi CBS 271.37, Klebsiella pneumoniae subsp. pneumoniae EcI8, Mycobacterium goodii, Paracoccus denitrificans, Penicillium brasilianum, Enterobacter sp. TL3, Aspergillus terreusNIH2624, Exophiala spinifera, Deinococcus geothermalis (strain DSM 11300), Geomyces destructans 20631-21 (GdTA) in E. coli, Pseudomonas putida KT2440, Lysinibacillus sphaericus, Bacillus megaterium DSM 319, Trichoderma harzianum, Aspergillus fumigatus R-ATAs (AspFum), Geobacillus thermodenitrificans subsp. thermodenitrificans DSM 465, Cladophialophora bantiana CBS 173.52, Bacillus megaterium, Burkholderia thailandensis MSMB121 ( BtS -TA ), Klebsiella pneumoniae subsp. pneumoniae MGH 78578, Geobacillus toebii, and Talaromyces cellulolyticus; the transaminase has the amino acid sequence of SEQ ID No: 1, SEQ ID No: 2, SEQ ID No: 3 or SEQ ID No: 4; the fluorine-containing dihydroxy ketal compound is selected from any one of the group consisting of:

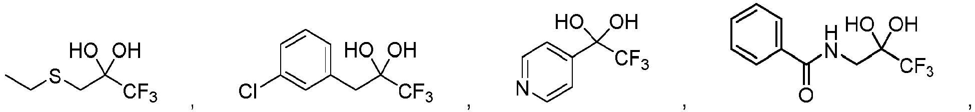

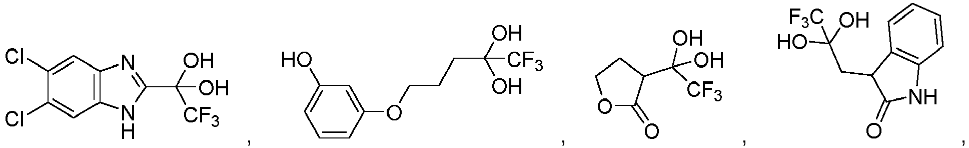

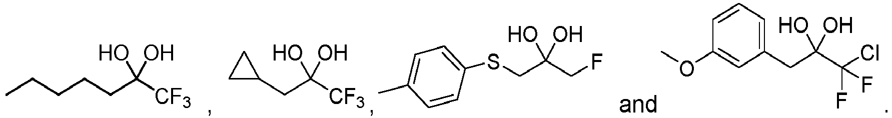

2. The method according to claim 1, wherein the method comprises:
mixing a phosphate buffer with the amino donor to obtain a first mixed solution;
adding the fluorine-containing dihydroxy ketal compound to the first mixed solution to obtain a second mixed solution;
adding the transaminase to the second mixed solution to obtain a reaction mixture;
isolating the fluorine-containing chiral amine compound from the reaction mixture.

3. The method according to claim 2, wherein before adding the fluorine-containing dihydroxy ketal compound to the first mixed solution, the method further comprises: adjusting a pH value of the first mixed solution to 7.0-9.0.

4. The method according to claim 3, wherein adding the transaminase to the second mixed solution, and adjusting a pH of the second mixed solution after addition of the transaminase to 7.0-9.0 to obtain the reaction mixture.

5. The method according to claim 2, wherein isolating the fluorine-containing chiral amine compound from the reaction mixture comprises:
adjust the acidity of the reaction mixture to denature the transaminase to obtain a denatured transaminase;
filtering and removing the denatured transaminase to obtain a filtrate;
extracting the filtrate to obtain the fluorine-containing chiral amine compound.

6. The method according to claim 5, wherein adjusting a pH value of the reaction mixture to 1-2.

7. The method according to claim 5, wherein the filtering is carried out with dichloromethane.

8. The method according to claim 5, wherein the extracting is carried out with dichloromethane.

9. The method according to claim 5, wherein the extraction is carried out 2-5 times, and after the extractions, a step of drying organic phases resulting from the extractions is further included.

10. The method according to claim 5, wherein
combining the organic phases obtained from the extractions to obtain an extract;
drying the extract to obtain a dried organic phase of product;
concentrating the dried organic phase of product to no fraction under a temperature less than 35°C and a pressure no more than - 0.06 Mpa to obtain the fluorine-containing chiral amine compound.

11. The method according to any one of claims 1 to 10, wherein a mass ratio of the transaminase to the fluorine-containing dihydroxy ketal compound is 0.4:1 to 1.0:1.

12. The method according to any one of claims 1 to 10, wherein a concentration of the fluorine-containing dihydroxy ketal compound is 60g / L to 100g / L.

13. The method according to any one of claims 1 to 10, wherein the amino donor is selected from any one of the group consisting of isopropylamine, isopropylamine hydrochloride, alanine, aniline and n-butylamine.
